# EUROPEAN PATENT APPLICATION

(11) **EP 4 534 523 A1**
(43) Date of publication of application: **09.04.2025**
(21) Application number: 23811099.3
(22) Date of filing: 24.05.2023
(51) Int. Cl.: C07C 217/84, C07C 213/10, A61K 31/137, A61P 35/00

(54) **CRYSTAL FORM OF ELACESTRANT DIHYDROCHLORIDE, PREPARATION METHOD THEREFOR, AND USE THEREOF**

(30) Priority: 25.05.2022 CN 202210578072; 26.05.2022 CN 202210589950
(71) Applicant: Crystal Pharmaceutical (Suzhou) Co., Ltd., Suzhou, Jiangsu 215123 (CN)
(72) Inventor: QIAN, Jiale, Suzhou, Jiangsu 215123 (CN); SHI, Jiaming, Suzhou, Jiangsu 215123 (CN)
(74) Representative: dompatent von Kreisler Selting Werner - Partnerschaft von Patent- und Rechtsanwälten mbB
(86) International application number: PCT/CN2023/096068
(87) International publication number: WO 2023/227029

(57) **Abstract**

The present disclosure relates to a novel crystalline form of Elacestrant (referred to as "Compound I") dihydrochloride and preparation methods thereof, pharmaceutical compositions containing the crystalline form, and uses of the crystalline form for preparing estrogen receptor depressant drugs and drugs for treating breast cancer with ER+, HER2-, ESR1 mutations.

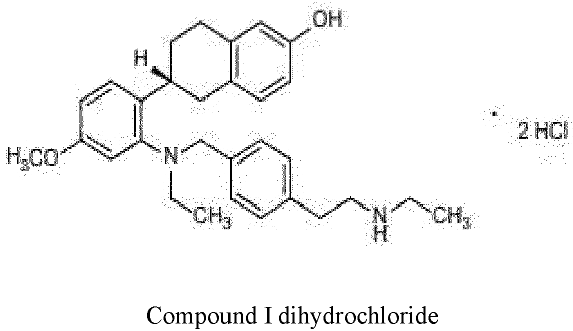

## Description

### TECHNICAL FIELD

The present disclosure pertains to the field of chemical crystallography, particularly relates to crystalline forms of Elacestrant, preparation method and use thereof.

### BACKGROUND

Breast cancer is cancer that develops from breast tissue. When breast cancer cells test estrogen receptor-positive, it's called estrogen receptor-positive (ER-positive) breast cancer. HER2 (human epidermal growth factor receptor 2) are receptors that sit on the surface of breast cells. When cancerous cells do not contain high levels of HER2, it's called HER2-negative breast cancer. At present, breast cancer is the most common cancer in the world, and its incidence rate ranks first among female malignant tumors. More than 2 million people are diagnosed every year in the world.

A new generation of oral estrogen receptor degrading agent (SERD) Elacestrant, jointly developed by Radius Health and Menarini Group, has been approved in the United States. It is for the treatment of postmenopausal women or adult men with advanced or metastatic breast cancer patients with ER+, HER2 -, ESR1 mutations who have previously received at least first-line endocrine therapy. Elacestrant has good selectivity towards estrogen receptors and has anti estrogen effects in different tissues.

The chemical name of Elacestrant is: (R)-6-{2-{ ethyl [4-(2-ethylaminoethyl)benzyl]amino }-4-methoxyphenyl}-5,6,7,8-tetrahydronaphthalen-2-ol, (Referred to as Compound I), and the structure is shown as the follows:

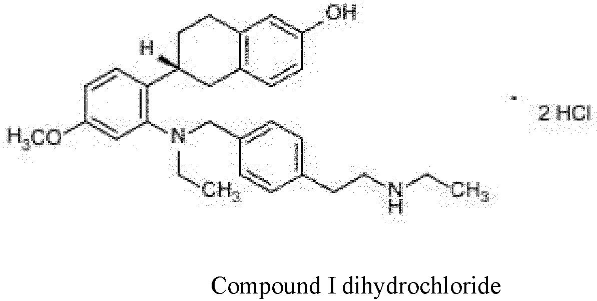

It is well known in the field that drug polymorphism is a common phenomenon in small molecule drug development and it is an important factor affecting drug quality. A crystalline form is a solid material whose constituents are arranged in a highly ordered microscopic structure, forming a crystal lattice that extends in all directions. Polymorphism refers to the phenomenon that a compound exists in more than one crystalline form. Compounds may exist in one or more crystalline forms, but their existence and characteristics cannot be predicted with any certainty. Different crystalline forms of drug substances have different physicochemical properties, including chemical stability, thermal stability, solubility, hygroscopicity and/or particle size, which can affect drug's in vivo dissolution and absorption and will further affect drug's clinical efficacy to some extent. In addition, different crystalline forms of drug substances have different manufacturing properties, including yield, purification properties, filtration properties, drying properties and milling properties, as well as stability with respect to pressure or compression forces during tableting, which may have an impact on the processing during the production of drug substances. Therefore, polymorphism is an important part of drug research and drug quality control.

At present, WO2018129419 disclosed Form 1, Form 2 and Form 3 of Compound I dihydrochloride. WO2020010216 disclosed Form 1B of Compound I dihydrochloride. WO2023064519 disclosed Form 6, Form 7 and Form 9 of Compound I dihydrochloride. Crystalline form CSII of the present disclosure is different from the above-mentioned forms. Figure 1 in WO2020010216 shows that Form 1 is converted to Form 3 under high humidity conditions. Form 2 is converted to Form 3 when humidity is below 40% RH. Form 3 transitions to Form 2 when humidity is above 40% RH. Table 17 in WO2020010216 disclosed that Form 1B can maintain stability at humidity above 90% RH. It indicates that Form 1B has the best stability among prior art crystalline forms.

Furthermore, step 6 of scheme 1 in WO2020167855 disclosed a preparation method for a crystalline form of Compound I dihydrochloride, but no XRPD data of the crystalline form were disclosed. The inventors of the present disclosure have repeated the preparation method to obtain the crystalline form, and it was confirmed to be Form 1B in prior art WO2020010216.

Based on the analysis of the prior arts mentioned above, it can be concluded that Form 1B is the best crystalline form in terms of properties. However, as disclosed in Table 17 of WO2020010216, Form 1B is a very small flaky particle mainly composed of aggregates. The particle size distribution of easily agglomerated crystalline forms are uneven, which may lead to uneven content of drug formulations. Furthermore, after in-depth research by the inventor of this disclosure, it was found that Form 1B still has disadvantages such as low solubility, poor flowability and compressibility, and high adhesiveness. Low solubility is not conducive to the absorption of drugs in vivo. Poor flowability and compressibility, as well as high adhesiveness, can result in uneven dispersion of raw materials and excipients during the mixing process. During the product tableting process, it is easy to occur molding failure, cracking and fragmentation, etc.

In order to overcome the disadvantages of prior arts, a novel crystalline form is still needed for the development of drugs containing Compound I dihydrochloride. The inventors of the present disclosure surprisingly discovered a crystalline form of Compound I dihydrochloride, which have advantages in at least one aspect of solubility, hygroscopicity, purification ability, stability, adhesiveness, compressibility, flowability, in vitro and in vivo dissolution, and bioavailability, etc. In particular, the crystalline form of the Compound I dihydrochloride of the present disclosure not only has good stability but also has advantages such as better solubility, purification ability, flowability, compressibility, adhesiveness, and uniform particle size distribution, which is of great significance for the development of drugs containing Compound I.

### SUMMARY

The present disclosure is to provide a novel crystalline form of Compound I dihydrochloride, preparation method and pharmaceutical compositions comprising the novel crystalline form.

According to the objective of the present disclosure, crystalline form CSII of Compound I dihydrochloride is provided (hereinafter referred to as Form CSII).

In one aspect provided herein, the X-ray powder diffraction pattern of Form CSII comprises characteristic peaks at 2theta values of 10.3°±0.2°, 12.1°±0.2° and 15.4°±0.2°using CuKα radiation.

Furthermore, the X-ray powder diffraction pattern of Form CSII comprises one or two or three or four characteristic peaks at 2theta values of 7.9°±0.2°, 9.2°±0.2°, 13.8°±0.2° and 11.0°±0.2°, using CuKα radiation; preferably, the X-ray powder diffraction pattern of Form CSII comprises characteristic peaks at 2theta values of 7.9°±0.2°, 9.2°±0.2°, 13.8°±0.2° and 11.0°±0.2° using CuKα radiation.

In other aspect provide herein, the X-ray powder diffraction pattern of Form CSII comprises three or four or five or six or seven or eight or nine or ten characteristic peaks at 2theta values of 10.3°±0.2°, 12.1°±0.2°, 15.4°±0.2°, 7.9°±0.2°, 9.2°±0.2°, 13.8°±0.2°, 11.0°±0.2°, 11.5°±0.2°, 17.4°±0.2° and 21.8°±0.2°, using CuKα radiation.

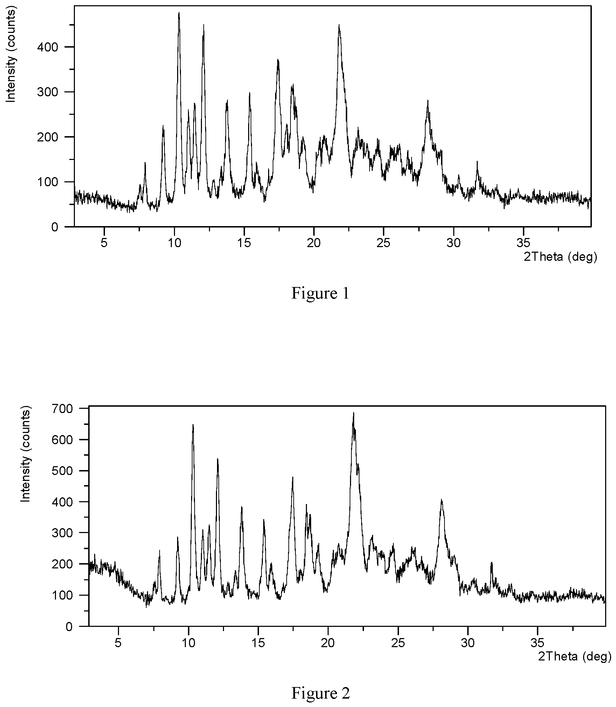

Without any limitation being implied, the X-ray powder diffraction pattern of Form CSII is substantially as depicted in Figure 1 or Figure 2 using CuKα radiation.

Without any limitation being implied, Form CSII is an anhydrate.

According to the objective of the present disclosure, a process for preparing Form CSII is also provided. The process comprises:
Adding Compound I dihydrochloride into chloroform, stirring, separating and drying to obtain Form CSII.

Said stirring temperatures is preferably 50 °C; said stirring is preferably more than 24 hours.

According to the objective of the present disclosure, the present disclosure also provides the use of Form CSII for preparing other crystalline forms, salts or cocrystals of Compound I.

According to the objective of the present disclosure, a pharmaceutical composition is provided, said pharmaceutical composition comprises a therapeutically effective amount of Form CSII and pharmaceutically acceptable excipients.

According to the objective of the present disclosure, Form CSII of present disclosure can be used for preparing estrogen receptor depressant drugs.

According to the objective of the present disclosure, Form CSII of present disclosure can be used for preparing drugs treating breast cancer with ER+, HER2-, ESR1 mutations.

### Advantages and technical problems solved by the present disclosure

The technical problem solved by the present disclosure is to provide a novel crystalline form different from the crystalline forms in prior arts. Based on excellent stability, the crystalline form also has higher solubility, better flowability, better compressibility, lower adhesiveness, better purification effect, no agglomeration and more uniform particle size distribution compared with prior art Form 1B, which solves the problems existing in prior arts.

Form CSII of the present disclosure has the following unexpected beneficial effects:
(1) Compared with prior art Form 1B, Form CSII of the present disclosure has better solubility. Especially in FeSSIF, the solubility is more than twice that of the prior art Form 1B.
   Elacestrant is a poorly water-soluble drug and belongs to BCS class IV, which means it is a drug with low solubility and low permeability, which are difficult to release and absorb in vivo and have low bioavailability. Higher solubility of Form CSII drug substance provided by the present disclosure is beneficial to improve drug's in vivo absorption.
(2) Compared with prior art Form 1B, Form CSII of the present disclosure has better purification effect. The purity is significantly increased after the raw material is converted into Form CSII of the present disclosure. In a specific embodiment, the purity of raw material is 99.14%. The purity of Form CSII made from the raw material is 99.80%. The purity is increased by 0.60%. Especially for impurities with RRT of 0.84, 1.14 and 1.43, Form CSII has significant purification ability. Chemical purity is of great significance for ensuring drug efficacy, safety and preventing the occurrence of adverse effects. As Form CSII of the present disclosure has good purification ability and is excellent in removing impurities. Thus drug substances with high purity can be obtained through crystallization, which effectively overcome the disadvantages of poor stability, poor efficacy and high toxicity caused by the low purity drug substances.
(3) Compared with prior art Form 1B, Form CSII of the present disclosure has no agglomeration, and more uniform of particle size distribution. A uniform particle size helps to reduce solvent enrichment, improve product purity, reduce product residue, ensure the uniformity of drug formulation content, and reduce the variability of in vitro dissolution.
(4) Compared with prior art Form 1B, Form CSII of the present disclosure has better flowability. Flowability evaluation results indicate that the flowability of Form CSII is remarkably better than that of prior art Form 1B. Better flowability can prevent clogging of production equipment and increase manufacturing efficiency. Better flowability of Form CSII ensures the content uniformity of the drug product, reduces the weight variation of the drug product and improves product quality.
(5) Compared with prior art Form 1B, Form CSII of the present disclosure has better compressibility. Failure in hardness/friability test and tablet crack issue can be avoided due to better compressibility, making the preparation process more reliable, improving product appearance, promoting product quality and production efficiency.
(6) Compared with prior art Form 1B, Form CSII of the present disclosure shows lower adhesiveness. Adhesiveness evaluation results indicate that adhesion quantity of Form CSII is remarkably lower than that of prior art forms. Low adhesiveness of Form CSII can reduce the agglomeration of drug substances and effectively improve the adhesion to roller and tooling during dry-granulation and compression process. It is conducive to the dispersion of drug substance with excipients and improving the blend uniformity of the mixing of materials, which ultimately improves product quality.
(7) Form CSII of the present disclosure has good physical stability under mechanical force. The crystalline form of Form CSII doesn't change after grinding and formulation process. Grinding and pulverization are often required in the drug manufacturing process. Good physical stability of the drug substance can reduce the risk of crystallinity decrease and crystal transformation during the drug production process. Furthermore, Form CSII has good physical stability under different pressures, which is beneficial to keep crystalline form unchanged during tableting process.
(8) From CSII drug substance and drug product of the present disclosure have good stability. Crystalline state of Form CSII drug substance doesn't change for at least 6 months when stored under the condition of 25 °C/60%RH. The chemical purity remains substantially unchanged during storage. After Form CSII is mixed with the excipients to form a drug product and stored under the condition of 25 °C/60%RH, crystalline state of Form CSII drug product doesn't change for at least 3 months. These results show that From CSII drug substance of the present disclosure has good stability under long term condition both itself and in drug product, which is beneficial to the drug storage. Good physical and chemical stability of drug substances ensures that no crystal transformation or impurities is generated during production and storage. Form CSII has good physical and chemical stability, ensuring consistent and controllable quality of the drug substance and drug product, minimizing quality change, bioavailability change and toxicity due to crystal transformation or impurity generation.

Meanwhile, crystalline state of Form CSII drug substance doesn't change for at least 6 months when stored under the condition of 40 °C/75% RH. The chemical purity remains substantially unchanged during storage. After Form CSII is mixed with the excipients to form a drug product and stored under the condition of 40 °C/75%RH, crystalline state of Form CSII drug product doesn't change for at least 1 month. The chemical purity remains substantially unchanged during storage. These results show that Form CSII drug substance and drug product have good stability under accelerated and stress conditions. High temperature and high humidity conditions caused by seasonal differences, climate differences in different regions and environmental factors will affect the storage, transportation and production of drug substances and preparations. Therefore, good stability under accelerated and stress conditions is of great importance to drug development.

### BRIEF DESCRIPTION OF THE DRAWINGS

Figure 1 shows an XRPD pattern of Form CSII
Figure 2 shows an XRPD pattern of Form CSII
Figure 3 shows a TGA curve of Form CSII
Figure 4 shows an XRPD pattern overlay of Form CSII before and after storage with different conditions (from top to bottom: initial, 25 °C/60%RH for 6 months sealed with desiccant and antioxidant, 40 °C/75%RH for 6 months sealed with desiccant and antioxidant).
Figure 5 shows an XRPD pattern overlay of Form CSII before and after ball milling
Figure 6 shows an XRPD pattern overlay of Form CSII before and after formulation process (from top to bottom: blank mixture, after formulation process, Form CSII.)
Figure 7 shows an XRPD pattern overlay of Form CSII drug product before and after storage with different conditions (from top to bottom: initial, 25 °C/60%RH for 3 months sealed with 1g of desiccant and 1g of antioxidant, 40 °C/75%RH for 1 month sealed with 1g of desiccant and 1g of antioxidant.)

### DETAILED DESCRIPTION

The present disclosure is further illustrated by the following examples which describe the preparation and use of the crystalline forms of the present disclosure in detail. It is obvious to those skilled in the art that changes in the materials and methods can be accomplished without departing from the scope of the present disclosure.

The abbreviations used in the present disclosure are explained as follows:
RH: Relative humidity
XRPD: X-ray Powder Diffraction
TGA: Thermo Gravimetric Analysis
HPLC: High Performance Liquid Chromatography
HDPE: High Density Polyethylene
FeSSIF: Fed-State Simulated Intestinal Fluid

Instruments and methods used for data collection:
XRPD patterns in the present disclosure were acquired by a Bruker X-ray powder diffractometer. The parameters of XRPD method of the present disclosure are as follows:
X-Ray source: Cu, Kα
Kα1 (Å): 1.54060; Kα2 (Å): 1.54439
Kα2/Kα1 intensity ratio: 0.50

TGA data in the present disclosure were acquired by a TA Q500. The parameters of the TGA method of the present disclosure are as follows:
Heating rate: 10 °C/ min
Purge gas: nitrogen

The particle size distribution data in the present disclosure were acquired by a Mastersizer 3000 laser particle size analyzer of Malvern. The test was carried out in wet mode, using a Hydro MV dispersion device, and the dispersant was Isopar G. The parameters are as follows:

| | |
|---|---|
| Size distribution: Volume | Scattering model: Mie |
| Dispersant name: Isopar G | Fluid refractive index: 1.420 |
| Number of measurements: 3 times | Measurement duration: 10 s |
| Absorption index: 0.100 | Particle refractive index: 1.520 |
| Particle type: irregularity | Analysis model: general |
| Stirrer speed: 2000 rpm | N/A |

The parameters for related substance and Compound I detection in the present disclosure are shown in Table 1.

**Table 1**

| | | |
|---|---|---|
| HPLC | Waters ACQUITY UPLC H-Class plus with PDA detector | |
| Column | Waters ACQUITY UPLC BEH Shield RP18, 2.1mm×50 mm,1.7 µm | |
| Mobile Phase | A: 0.1% H₃PO₄ in H₂O (pH2.5, TEA) | |
| | B: ACN: MeOH=800:200 (v/v) | |
| Gradient | Time (min) | %B |
| | 0.0 | 10 |
| | 9.0 | 40 |
| | 12.0 | 80 |
| | 16.0 | 80 |
| | 16.1 | 10 |
| | 18.0 | 10 |
| Flow rate | 0.5 mL/min | |
| Injection volume | 1 µL | |
| Detector wavelength | UV at 240 nm | |
| Column temperature | 40 °C | |
| Sample temperature | Room temperature | |
| Diluent | Acetonitrile: H₂O = 50:50 (v/v) | |

The parameters for kinetic solubility in the present disclosure are shown in Table 2.

**Table 2**

| | | |
|---|---|---|
| HPLC | Waters ACQUITY UPLC H-Class plus with PDA director | |
| Column | Waters ACQUITY UPLC BEH Shield RP18, 2.1mm×50 mm,1.7 µm | |
| Mobile Phase | A: 0.1% H₃PO₄ in H₂O (pH2.5, TEA) | |
| | B: ACN: MeOH=800:200 (v/v) | |
| Gradient | Time (min) | %B |
| | 0.0 | 10 |
| | 9.0 | 40 |
| | 12.0 | 80 |
| | 13.0 | 80 |
| | 13.1 | 10 |
| | 15.0 | 10 |
| Flow rate | 0.5 mL/min | |
| Injection volume | 1 µL | |
| Detector wavelength | 215 nm | |
| Column temperature | 40 °C | |
| Sample temperature | Room temperature | |
| Diluent | Acetonitrile: H₂O=50:50 (v/v) | |

The parameters for the content of chloride ion in the present disclosure are shown in Table 3.

**Table 3**

| | |
|---|---|
| IC | Thermo Fisher Dionex Aquion |
| Column | Thermo Dionex IonPac AS22, 4×250 mm, 6.0 µm |
| Eluent | 4.5mM Na₂CO₃/1.4mM NaHCO₃ |
| Injection volume | 25 µL |
| Flow rate | 1.0 mL/min |
| Conductivity cell temperature | 35 °C |
| Column temperature | 30 °C |
| Current | 31 mA |
| Run time | 8-10 min |

Said "stirring" is accomplished by using a conventional method in the field such as magnetic stirring or mechanical stirring and the stirring speed is 50 to 1800 r/min. Preferably the magnetic stirring speed is 300 to 900 r/min and mechanical stirring speed is 100 to 300 r/min.

Said "separation" is accomplished by using a conventional method in the field such as centrifugation or filtration. The operation of "centrifugation" is as follows: the sample to be separated is placed into the centrifuge tube, and then centrifuged at a rate of 10000 r/min until the solid all sinks to the bottom of the tube.

Said "drying" is accomplished by using a conventional method in the field such as vacuum drying, blast drying or free-air drying. The drying temperature can be room temperature or higher. Preferably the drying temperature is from room temperature to about 60 °C, or to 50 °C, or to 40 °C. The drying time can be 2 to 48 hours, or overnight. Drying is accomplished in a fume hood, forced air convection oven or vacuum oven.

Said "characteristic peak" refers to a representative diffraction peak used to distinguish crystals, which usually can have a deviation of ±0.2° using CuKα radiation.

In the present disclosure, "crystal" or "crystalline form" refers to the crystal or the crystalline form being identified by the X-ray diffraction pattern shown herein. Those skilled in the art are able to understand that the X-ray powder diffraction pattern depend on the instrument conditions, the sample preparation and the purity of samples. The relative intensity of the diffraction peaks in the X-ray diffraction pattern may also vary with the experimental conditions; therefore, the order of the diffraction peak intensities cannot be regarded as the sole or decisive factor. In fact, the relative intensity of the diffraction peaks in the X-ray powder diffraction pattern is related to the preferred orientation of the crystals, and the diffraction peak intensities shown herein are illustrative and identical diffraction peak intensities are not required. Thus, it will be understood by those skilled in the art that a crystalline form of the present disclosure is not necessarily to have exactly the same X-ray diffraction pattern of the example shown herein. Any crystalline forms whose X-ray diffraction patterns have the same or similar characteristic peaks should be within the scope of the present disclosure. Those skilled in the art can compare the patterns shown in the present disclosure with that of an unknown crystalline form in order to identify whether these two groups of patterns reflect the same or different crystalline forms.

In some embodiments, Form CSII of the present disclosure is pure and substantially free of any other crystalline forms. In the present disclosure, the term "substantially free" when used to describe a novel crystalline form, it means that the content of other crystalline forms in the novel crystalline form is less than 20% (w/w), specifically less than 10% (w/w), more specifically less than 5% (w/w) and furthermore specifically less than 1% (w/w).

In the present disclosure, the term "about" when referring to a measurable value such as weight, time, temperature, and the like, is meant to encompass variations of ± 10%, ± 5%, ± 1%, ± 0.5%, or even ± 0.1% of the specified amount.

Unless otherwise specified, the following examples were conducted at room temperature and environment humidity. Said "room temperature" is not a specific temperature, but a temperature range of 10-30 °C. Said "environment humidity" is not a specific humidity, but a humidity range of 10%RH-90%RH, 20%RH-80%RH or 30%RH-70%RH.

According to the present disclosure, Compound I and/or its salt used as raw materials include, but are not limited to solid (crystalline and amorphous), semisolid, wax, oil, liquid form or solution. Preferably, Compound I and/ or its salt used as the raw material is a solid.

Raw materials of Compound I and/or salts thereof used in the following examples were prepared by known methods in prior arts, for example, the method disclosed in WO2018129419.

### Example 1: Preparation of Form CSII

10.6 mg of Compound I dihydrochloride was weighed into a glass vial, and 0.1 mL of chloroform was added thereafter. The suspension was stirred at 50 °C for 21 days, then tranferred to -20 °C and kept for 6 days. The solid was seperated and dried under vacuum overnight to obtain a dry solid.

The dry solid was confirmed to be Form CSII by XRPD. The XRPD pattern is substantially as depicted in Figure 1, and the XRPD data are listed in Table 4.

**Table 4**

| 2θ (°) | d-spacing (Å) | Intensity (%) |
|---|---|---|
| 7.57 | 11.68 | 11.90 |
| 7.91 | 11.17 | 23.43 |
| 9.21 | 9.60 | 39.27 |
| 10.33 | 8.56 | 100.00 |
| 11.01 | 8.04 | 50.43 |
| 11.46 | 7.72 | 53.27 |
| 12.09 | 7.32 | 89.37 |
| 12.83 | 6.90 | 13.06 |
| 13.76 | 6.43 | 52.55 |
| 15.39 | 5.76 | 58.61 |
| 15.91 | 5.57 | 18.80 |
| 17.42 | 5.09 | 72.23 |
| 18.02 | 4.92 | 39.62 |
| 18.44 | 4.81 | 58.26 |
| 18.74 | 4.73 | 48.77 |
| 19.26 | 4.61 | 33.06 |
| 20.29 | 4.38 | 26.21 |
| 20.74 | 4.28 | 33.38 |
| 21.80 | 4.08 | 91.98 |
| 23.22 | 3.83 | 32.66 |
| 24.57 | 3.62 | 27.99 |
| 26.05 | 3.42 | 27.24 |
| 26.77 | 3.33 | 20.99 |
| 28.11 | 3.17 | 50.24 |
| 28.98 | 3.08 | 24.03 |
| 30.36 | 2.94 | 10.83 |
| 31.69 | 2.82 | 17.08 |
| 32.94 | 2.72 | 4.01 |

### Example 2: Preparation of Form CSII

99.7 mg of Compound I dihydrochloride was weighed into a glass vial, and 1 mL of chloroform was added thereafter. An appropriate amount of Form CSII seeds was added. The suspension was stirred for 3 days at 50 °C, then tranferred to room temperature and dried under vacuum for 2 days to obtain a dry solid.

The dry solid was confirmed to be Form CSII by XRPD. The XRPD pattern is substantially as depicted in Figure 2, and the XRPD data are listed in Table 5.

**Table 5**

| 2θ (°) | d-spacing (Å) | Intensity (%) |
|---|---|---|
| 7.57 | 11.68 | 7.41 |
| 7.91 | 11.17 | 23.56 |
| 9.24 | 9.57 | 35.19 |
| 10.33 | 8.56 | 98.60 |
| 11.03 | 8.02 | 39.29 |
| 11.48 | 7.71 | 42.26 |
| 12.09 | 7.32 | 79.28 |
| 12.84 | 6.89 | 8.55 |
| 13.36 | 6.63 | 15.41 |
| 13.80 | 6.42 | 52.22 |
| 15.42 | 5.75 | 41.78 |
| 15.93 | 5.57 | 18.59 |
| 17.45 | 5.08 | 68.10 |
| 18.46 | 4.81 | 48.92 |
| 18.75 | 4.73 | 41.93 |
| 19.27 | 4.61 | 28.72 |
| 20.33 | 4.37 | 23.38 |
| 20.75 | 4.28 | 30.90 |
| 21.78 | 4.08 | 100.00 |
| 22.24 | 4.00 | 64.39 |
| 23.11 | 3.85 | 33.62 |
| 23.81 | 3.74 | 24.86 |
| 24.64 | 3.61 | 28.93 |
| 26.09 | 3.42 | 26.45 |
| 28.14 | 3.17 | 54.52 |
| 29.01 | 3.08 | 23.46 |
| 30.41 | 2.94 | 9.56 |
| 31.68 | 2.82 | 21.10 |
| 33.04 | 2.71 | 5.00 |

### Example 3 TGA curve of Form CSII

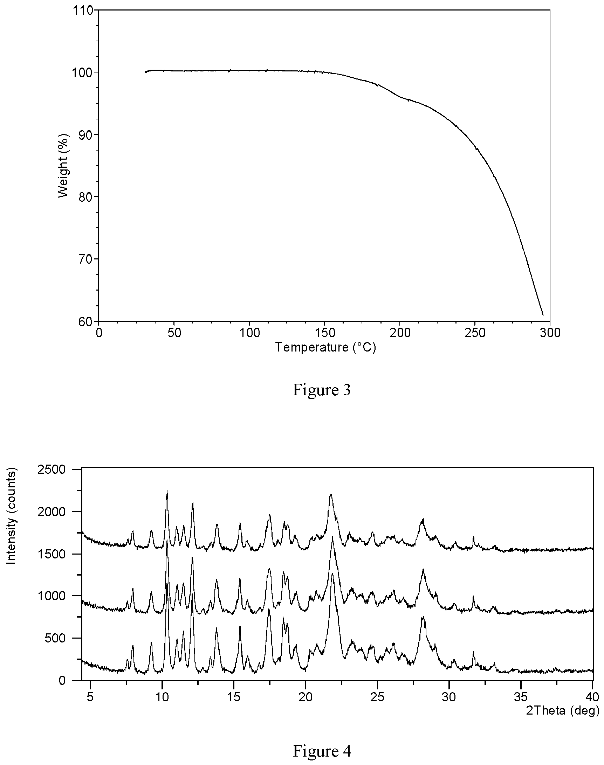

The TGA curve of Form CSII is substantially as depicted in Figure 3, and it shows Form CSII almost has no weight loss when heated to 100°C.

### Example 4 Content of chloride ion of Form CSII

The content of chloride ion and free base of Form CSII of the present disclosure was tested, and the molar ratio was calculated. The results are shown in Table 6. The results indicate that the molar ratio of Form CSII of the present disclosure is 2.0 and Form CSII is a dihydrochloride of Compound I.

**Table 6**

| Form | Molar ratio (chloride ion: free base) |
|---|---|
| Form CSII | 2.03 |

### Example 5 Kinetic solubility of Form CSII

When solubility test is used to predict the in vivo performance of a drug, it is critical to simulate in vivo conditions as closely as possible. For oral medication, Fed-State Simulated Intestinal Fluid (FeSSIF) can be used to simulate the condition in vivo and predict the effects of feeding, thus solubility in such mediums is closer to that in vivo.

10 mg of Form CSII and 10 mg of prior art Form 1B were suspended into 1 mL of FeSSIF to get suspensions. After equilibrated at 37 °C for 30 min, the suspensions were filtrated to get saturated solutions. The concentrations (mg/mL) of Compound I of the saturated solutions were measured by HPLC. The results are listed in Table 7. The results show that the solubility of Form CSII in FeSSIF is higher than that of Form 1B, and the solubility of Form CSII is 2.2 times that of Form 1B.

**Table 7**

| Medium | Solubility (mg/mL) | |
|---|---|---|
| | Prior art Form 1B | Form CSII |
| FeSSIF | 1.17 | 2.59 |

### Example 6 Purification ability of Form CSII

Form CSII and prior art Form 1B were prepared with the same starting material. HPLC was applied to test the chemical purity of starting material, Form CSII and prior art Form 1B. The results are listed in Table 8. The results show that chemical purity of Form CSII is higher than that of prior art Form 1B when the same starting material was used, especially for impurities with RRT (relative retention time) of 0.84, 1.14 and 1.43. Form CSII has better purification ability than prior art Form 1B, and the impurity content is below the detection limit.

**Table 8**

| Form | Purity | Purity increment | RRT=0.84 | RRT=1.14 | RRT=1.43 |
|---|---|---|---|---|---|
| Starting material | 99.20% | - | 0.04% | 0.08% | 0.11% |
| Form CSII | 99.80% | 0.60% | ND | ND | ND |
| Prior art Form 1B | 99.67% | 0.47% | 0.05% | 0.05% | 0.04% |

| | | | | | |
|---|---|---|---|---|---|
| ND: Not detected | | | | | |

### Example 7 Morphology of Form CSII

An appropriate amount of Form CSII of the present disclosure was placed on a glass slide and dispersed with vacuum pump oil. It was covered with a cover glass slide and shoot at a magnification of 200 times. The results indicate that Form CSII has no agglomeration.

### Example 8 Particle size distribution of Form CSII

An appropriate amount of Form CSII of the present disclosure and prior art Form 1B was tested particle size distribution. The average particle diameter calculated by volume, the diameter at which 10% mass is comprised of smaller particles (D10), the diameter at which 50% mass is comprised of smaller particles (D50) and the diameter at which 90% mass is comprised of smaller particles (D90) were obtained in particle size distribution test. The results are shown in Table 9. The results show that the particle size distribution of Form CSII is uniform, which is superior to that of prior art Form 1B.

**Table 9**

| Form | MV (µm) | D10 (µm) | D50 (µm) | D90 (µm) |
|---|---|---|---|---|
| Form CSII | 15.8 | 2.11 | 14.9 | 28.3 |
| Prior art Form 1B | 350 | 1.64 | 286 | 839 |

### Example 9 Flowability of Form CSII

Compressibility index is usually utilized to evaluate the flowability of powder or granules during the drug product process. Compressibility index test method is as follows: a certain amount of powder was added into a measuring cylinder and bulk volume was recorded. Then the powder was tapped to make it in the tightest state and the tapped volume was recorded. The bulk density (ρ₀), tapped density (ρ_{f}) were calculated and compressibility index was calculated according to c=(ρ_{f} - ρ₀)/ρ_{f}. According to the criteria of flowability in USP General Charpters: <1174> Powder Flow, the flowability results are shown in Table 10. The results indicate that flowability of Form CSII is remarkably superior to that of the prior art Form 1B.

**Table 10**

| Form | Bulk density (g/m) | Tapped density (g/mL) | Compressibility index (%) | Flowability |
|---|---|---|---|---|
| Prior art Form 1B | 0.198 | 0.248 | 20 | Fair |
| Form CSII | 0.237 | 0.266 | 11 | Good |

### Example 10 Compressibility of CSII

An ENERPAC manual tablet press was used for compression. 60 mg of Form CSII and prior art Form 1B were weighed and added into the dies of a ϕ6mm round tooling, compressed at 5 KN manually, then stored at room temperature for 24 h until elastic recovery is complete, hardness (H) was tested with an intelligent tablet hardness tester. Diameter (D) and thickness (L) were tested with a caliper. Tensile strength of the powder was calculated with the following formula: *T=2H*/*πDL.* Under a certain force, the greater the tensile strength, the better the compressibility. The results are presented in Table 11. The results indicate that Form CSII has better compressibility compared with prior art Form 1B.

**Table 11**

| Form | Hardness (kgf) | Diameter (mm) | Thickness (mm) | Tensile strength (MPa) |
|---|---|---|---|---|
| Prior art Form 1B | 9.3 | 6.06 | 2.21 | 0.44 |
| Form CSII | 18.4 | 6.04 | 2.00 | 0.97 |

### Example 11 Adhesiveness of Form CSII

30 mg of Form CSII and 30 mg of prior art Form 1B were weighed and then added into the dies of Φ8mm round tooling, compressed at 10 KN by ENERPAC manual tablet press and held for 30s. The punch was weighed and amount of material sticking to the punch was calculated. The compression was repeated twice and the average amount of material sticking to the punch during the compression were calculated. Detailed experimental results are shown in Table 12. Test results indicate that the adhesiveness of Form CSII is lower than that of prior art Form 1B.

**Table 12**

| Form | Average amount (mg) |
|---|---|
| Prior art Form 1B | 0.59 |
| Form CSII | 0.21 |

### Example 12 Stability of Form CSII

An appropriate amount of Form CSII of the present disclosure were stored under different sealed conditions of 25 °C/60%RH and 40 °C/75%RH with desiccant and antioxidant. Chemical purity and crystalline form were checked by HPLC and XRPD, respectively. The results are shown in Table 13, and the XRPD overlay is shown in Figure 4. The results show that Form CSII kept stable for at least 6 months at sealed conditions of 25 °C/60%RH and 40 °C/75%RH. Form CSII has good stability under both long-term and accelerated conditions.

**Table 13**

| Initial | Condition | Package | Time | Form | Purity |
|---|---|---|---|---|---|
| Form CSII | Initial | - | - | Form CSII | 99.59% |
| | 25°C/60%RH | Sealed with desiccant and antioxidant | 6 months | Form CSII | 99.60% |
| | 40°C/75%RH | | 6 months | Form CSII | 99.58% |

### Example 13 Stability of Form CSII upon mechanical force

Approximately 20 mg of Form CSII was added into the dies of a Φ6mm round tooling, compressed into tablets under different pressures. Crystalline forms before and after tableting were checked by XRPD. The test results are shown in Table 14. The results show that Form CSII has better stability under different pressures.

**Table 14**

| Before tableting | Pressure | Solid form after tableting |
|---|---|---|
| Form CSII | 5 kN | Form CSII |
| | 10 kN | Form CSII |
| | 15 kN | Form CSII |

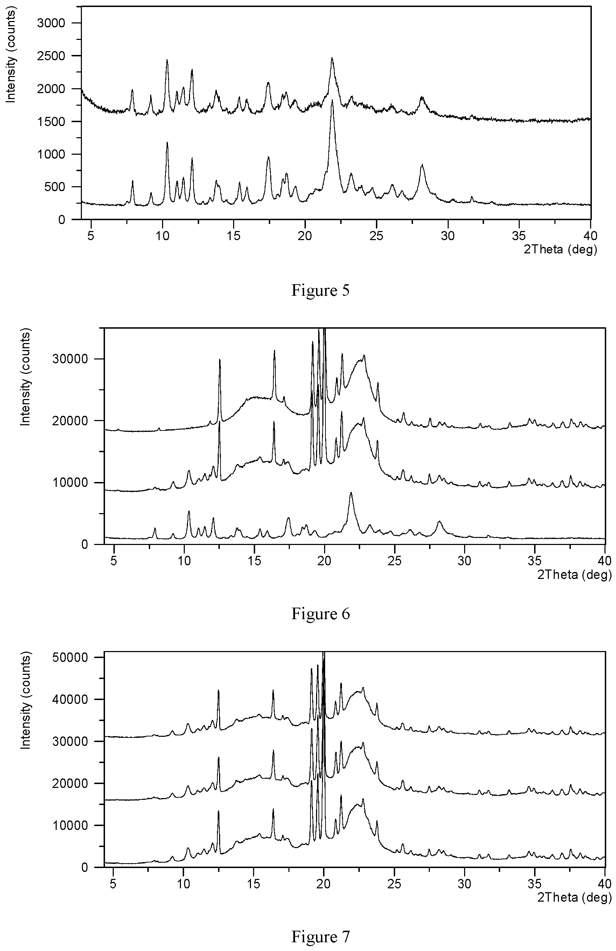

Approximately 20 mg of Form CSII of the present disclosure was placed in a centrifuge tube, then zirconium beads were added. Ball milling at 500 rpm for 10 minutes. Crystalline forms before and after ball milling were checked by XRPD. The test results are shown in Figure 5. The results show that Form CSII does not change after ball milling, and the grinding stability is good.

### Example 14 Preparation of Form CSII drug product

Form CSII of the present disclosure was made into capsules using the formulation in Table 15 and the preparation process in Table 16. The XRPD patterns were collected before and after the formulation process. The XRPD overlay is shown in Figure 6. The results indicate that Form CSII remains stable before and after the formulation process.

**Table 15**

| No. | Component | mg/unit | % (w/w) |
|---|---|---|---|
| 1 | Compound I dihydrochloride (Form CSII) | 23.2* | 23.2* |
| 2 | Microcrystalline Cellulose | 51.4 | 51.4 |
| 3 | Lactose | 19.4 | 19.4 |
| 4 | Sodium Carboxymethyl Starch | 5 | 5 |
| 5 | Magnesium stearate | 0.5 | 0.5 |
| 6 | Colloidal silicon dioxide | 0.5 | 0.5 |
| Total | | 100 | 100 |
| Note: The mass of 23.2 mg of Compound I dihydrochloride corresponds to 20 mg of Compound I free base. | | | |

**Table 16**

| Stage | Procedure |
|---|---|
| Pre-blending | According to the formulation, materials No. 1-6 were weighed into an LDPE bags and blended for 2 mins. |
| Simulation of dry granulation | The mixture was pressed by a single punch manual tablet press (type: ENERPAC, die: Φ 20 mm round, pressure: 5 ± 1 KN). The flakes were pulverized and sieved through a 20-mesh sieve. |
| Tableting | The total mixed powder (approximately 100 mg) was added into a T 8×4 punch die and compressed at 5 KN to obtain a tablet. |
| Package | A tablet was sealed in a 35 cc HDPE bottle with 1 g of desiccant and 1 g of antioxidant. |

### Example 15 Stability of Form CSII drug product

The Form CSII drug product was stored under sealed conditions of 25 °C/60%RH and 40 °C/75%RH with 1 g of desiccant and 1 g of antioxidant, and the crystalline forms and purity before and after storage were tested to evaluate its stability. The results are shown in Table 17 and the XRPD pattern overlay is shown in Figure 7. The results show that Form CSII drug product kept stable for at least 3 months at 25 °C/60%RH and at least 1 month at 40 °C/75%RH conditions, which indicate Form CSII drug product has good stability under both long-term and accelerated conditions.

**Table 17**

| Initial | Condition | Package | Time | Form | Purity |
|---|---|---|---|---|---|
| Form CSII | Initial | - | - | Form CSII | 99.64% |
| | 25°C/60%RH | Sealed with desiccant and antioxidant | 3 months | Form CSII | 99.60% |
| | 40°C/75%RH | | 1 month | Form CSII | 99.60% |

The examples described above are only for illustrating the technical concepts and features of the present disclosure and intended to make those skilled in the art being able to understand the present disclosure and thereby implement it and should not be concluded to limit the protective scope of this disclosure. Any equivalent variations or modifications according to the spirit of the present disclosure should be covered by the protective scope of the present disclosure.

## Claims

1. Crystalline form of Compound I dihydrochloride, wherein the X-ray powder diffraction pattern comprises characteristic peaks at 2theta values of 10.3°±0.2°, 12.1°±0.2° and 15.4°±0.2° using CuKα radiation

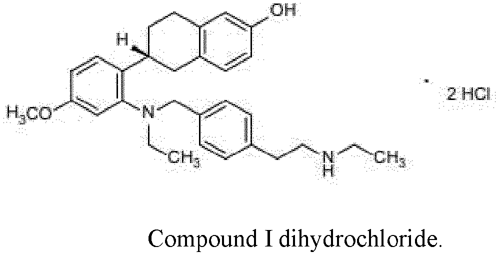

2. The crystalline form of Compound I dihydrochloride according to claim 1, wherein the X-ray powder diffraction pattern comprises at least one characteristic peaks at 2theta values of 7.9°±0.2°, 9.2°±0.2°, 13.8°±0.2° and 11.0°±0.2° using CuKα radiation.

3. The crystalline form of Compound I dihydrochloride according to claim 1, wherein the X-ray powder diffraction pattern of the crystalline form is substantially as depicted in Figure 1 or Figure 2 using CuKα radiation.

4. The crystalline form of Compound I dihydrochloride according to claim 1, wherein the crystalline form is an anhydrate.

5. A pharmaceutical composition, wherein said pharmaceutical composition comprises a therapeutically effective amount of crystalline form of Compound I dihydrochloride according to claim 1, and pharmaceutically acceptable excipients.
